# EUROPEAN PATENT APPLICATION

(11) **EP 4 741 500 A1**
(43) Date of publication of application: **13.05.2026**
(21) Application number: 24817475.7
(22) Date of filing: 07.06.2024
(51) Int. Cl.: C12N 15/11, C12N 15/113, A61K 31/711

(54) **POLYNUCLEOTIDE, PHARMACEUTICAL COMPOSITION, AND USE THEREOF**

(30) Priority: 09.06.2023 CN 202310688123
(71) Applicant: Liangzhu Laboratory, Hangzhou, Zhejiang 310000 (CN)
(72) Inventor: SHEN, Ning, Hangzhou, Zhejiang 310000 (CN); LIANG, Jinzhao, Hangzhou, Zhejiang 310000 (CN); LV, Lin, Hangzhou, Zhejiang 310000 (CN)
(74) Representative: Maiwald GmbH
(86) International application number: PCT/CN2024/098005
(87) International publication number: WO 2024/251244

(57) **Abstract**

The present application discloses a polynucleotide, a pharmaceutical composition and uses thereof. In the present application, various novel polynucleotide sequences targeting the 3' untranslated region of lamin A mutant mRNA are designed, which can reduce the expression level of lamin A mutants (e.g., progerin) at the Lamin A mRNA stage, while not affecting the normal transcription of Lamin C, thereby resulting in reduced toxicity. These sequences show broad prospects in the prevention or treatment of diseases associated with lamin A mutations, particularly progeria.

## Description

This application claims priority to Chinese Patent Application No. CN202310688123.8, filed on June 9, 2023, entitled "Polynucleotide, Pharmaceutical Composition and Uses Thereof".

### TECHNICAL FIELD

The present application relates to the field of biomedicine, and specifically to a polynucleotide, a pharmaceutical composition and uses thereof having efficacy in the treatment of certain diseases.

### BACKGROUND

Lamin A protein is a component of the nuclear lamina structure and plays an important role in nuclear structure and function. Mutations in the LMNA gene can disrupt peripheral chromatin with specific epigenetic and molecular characteristics, leading to the erroneous expression of genes normally expressed in other cell types, causing congenital diseases such as progeria, familial dilated cardiomyopathy, muscular dystrophy, and lipodystrophy syndrome.

Hutchinson-Gilford Progeria Syndrome, also known as childhood progeria, is a rare and fatal genetic disease. The incidence is approximately one in every 4 to 8 million newborns worldwide. HGPS is characterized by an accelerated aging process that is 5 to 10 times faster than normal. Patients exhibit a premature aging appearance resembling elderly individuals, accompanied by rapid systemic organ deterioration and a decline in physiological functions. Clinical symptoms include short stature, alopecia (hair loss), and delayed tooth eruption. Most patients die between the ages of 7 and 20, primarily from age-related diseases, such as cardiovascular disease.

Existing studies have documented the possible cause of progeria, which is caused by a mutation in the *LMNA* gene encoding lamin A that results in a 150-nucleotide deletion in exon 11 containing the cleavage site for the zinc metalloproteinase 24 (ZMPSTE24) enzyme, preventing the normal cleavage of the farnesylated C-terminus of Lamin A. This leads to the production of a permanently farnesylated, toxic protein called progerin, which is the cause of the gradual degeneration of cell structure and function in children with progeria. Mutations occurring in the ZMPSTE24 gene can also lead to the formation of permanently farnesylated Lamin A protein, causing similar aging symptoms.

Currently, Lonafarnib (trade name: Zokinvy) is the only FDA-approved drug for progeria. Lonafarnib is an oral farnesyltransferase inhibitor involved in protein isoprenylation modification. By inhibiting the isoprenylation of progerin, Lonafarnib can reduce the accumulation of progerin in the nucleus. However, clinical studies indicate that Lonafarnib does not restore a normal lifespan and is associated with safety concerns. Therefore, there remains a need for safer and more effective drugs for treating progeria.

### SUMMARY OF THE INVENTION

To address and/or overcome the technical problems existing in the background art, an object of the present application is to provide a polynucleotide.

Another object of the present application is to provide a pharmaceutical composition.

Another object of the present application is to provide uses of the aforementioned polynucleotide or pharmaceutical composition.

Another object of the present application is to provide a method for preventing and/or treating diseases associated with lamin A mutations.

To achieve the above technical objectives, a first aspect of the present application provides a polynucleotide, said polynucleotide comprising a polynucleotide fragment complementary to the following sequence or a fragment thereof:
(i) the sequence shown in SEQ ID NO: 11; and/or
(ii) a polynucleotide sequence formed by substitution, deletion, or addition of one or several nucleotides in the sequence shown in SEQ ID NO: 11.

In some preferred embodiments, said polynucleotide comprises a polynucleotide fragment complementary to the following sequence or a fragment thereof:
(i) the sequence shown in SEQ ID NO: 1; and/or
(ii) a polynucleotide sequence formed by substitution, deletion, or addition of one or several nucleotides in the sequence shown in SEQ ID NO: 1.

In some preferred embodiments, said polynucleotide comprises a polynucleotide fragment complementary to the following sequence or a fragment thereof:
(i) the sequence shown in SEQ ID NO: 10; and/or
(ii) a polynucleotide sequence formed by substitution, deletion, or addition of one or several nucleotides in the sequence shown in SEQ ID NO: 10.

In some preferred embodiments, said polynucleotide comprises at least one polynucleotide fragment selected from the group consisting of:
(i) the polynucleotide fragments shown in SEQ ID NOs: 2-9; and
(ii) polynucleotide fragments having at least 80% sequence identity with SEQ ID NOs: 2-9.

In some preferred embodiments, said polynucleotide is at least one selected from the group consisting of:
(i) the polynucleotides shown in SEQ ID NOs: 2-9; and
(ii) polynucleotides having at least 80% sequence identity with SEQ ID NOs: 2-9.

In some preferred embodiments, said polynucleotide is the polynucleotide shown in SEQ ID NO: 5 or the polynucleotide shown in SEQ ID NO: 7.

In some preferred embodiments, the length of said polynucleotide is not less than 10 bp, further preferably not less than 15 bp, more preferably 18-22 bp, for example, 20 bp.

In some preferred embodiments, the length of said polynucleotide is not more than 100 bp.

In some preferred embodiments, said polynucleotide is obtained by isolation.

A second aspect of the present application provides a small nucleic acid molecule comprising the polynucleotide according to the first aspect of the present application, said small nucleic acid molecule being siRNA, ASO, shRNA, or miRNA.

A third aspect of the present application provides a pharmaceutical composition comprising the polynucleotide according to the first aspect of the present application and a pharmaceutically acceptable carrier, or the small nucleic acid molecule according to the second aspect of the present application and a pharmaceutically acceptable carrier.

A fourth aspect of the present application provides uses of the polynucleotide according to the first aspect of the present application, the small nucleic acid molecule according to the second aspect of the present application, or the pharmaceutical composition according to the third aspect of the present application, said uses comprising at least one of the following:
(i) preventing and/or treating a disease associated with a lamin A mutation;
(ii) preparing a medicament for preventing and/or treating a disease associated with a lamin A mutation;
(iii) reducing the expression level of a lamin A mutant (preferably reducing the expression level of progerin);
(iv) reducing the number of senescence-positive cells;
(v) reducing the number of cells with dysmorphic nuclei.

In some preferred embodiments, said lamin A mutation is a mutation in exon 11 and/or exon 12 of lamin A mRNA.

In some preferred embodiments, said disease associated with a lamin A mutation is a disease caused by a mutation in exon 11 and/or exon 12 of lamin A mRNA.

In some preferred embodiments, said disease associated with a lamin A mutation is a disease caused by a mutation in lamin A mRNA at one or more positions selected from: c.1711; c.1713; c.1714; c.1718; c.1733; c.1744; c.1745; c.1748; c.1751; c.1756; c.1762; c.1772; c.1774; c.1786; c.1804; c.1821; c.1822; c.1824; c.1851; c.1868; c.1871; c.1892; c.1904; c.1916; c.1928; c.1930; c.1931; c.1940; c.1960; c.1961; c.1968; c.1968+1; c.1968+2; c.1968+5; and c.1975.

In some preferred embodiments, said disease associated with a lamin A mutation is a disease caused by one or more mutations in lamin A selected from: c.1711A>T; c.1713C>A; c.1714A>T; c.1714insCTGC; c.1718C>T; c.1733A>T; c.1744C>T; c.1745G>A; c.1748C>T; c.1751G>A; c.1756G>A; c.1762T>C; c.1772G>T; c.1774G>A; c.1786G>A; c.1804G>A; c.1821G>A; c.1822G>A; c.1824C>T; c.1851C>T; c.1868C>G; c.1871G>A; c.1892G>A; c.1904G>A; c.1916A>G; c.1928C>A; c.1930C>T; c.1931G>A; c.1940T>G; c.1960C>T; c.1961dup; c.1968G>A; c.1968+1G>A; c.1968+2T>C; c.1968+5G>A; c.1968+5G>C; and c.1975dup.

In some preferred embodiments, said disease associated with a lamin A mutation is at least one selected from dilated cardiomyopathy and conduction disorders (DCM-CD), muscular dystrophy, Charcot-Marie-Tooth disease type 2 (CMT2), familial partial lipodystrophy (FPLD), striated muscle laminopathy, progeroid syndrome, Emery-Dreifuss muscular dystrophy (EDMD), insulin resistance syndrome (IRS), progeria, limb-girdle muscular dystrophy (LGMD), congenital muscular dystrophy (L-CMD) and Werner's syndrome, preferably progeria.

A fifth aspect of the present application provides a method for preventing and/or treating a disease associated with a lamin A mutation, said method comprising the step of:
administering a therapeutically effective amount of the polynucleotide according to the first aspect of the present application, the small nucleic acid molecule according to the second aspect of the present application, or the pharmaceutical composition according to the third aspect of the present application to a subject.

A sixth aspect of the present application provides a non-therapeutic in vitro method for (i) reducing the expression level of a Lamin A mutant, or (ii) reducing the number of senescence-positive cells, or (iii) reducing the number of cells with dysmorphic nuclei, said method comprising the step of:
contacting a biological tissue in a non-therapeutic manner in vitro with the polynucleotide according to the first aspect of the present application, the small nucleic acid molecule according to the second aspect of the present application, or the pharmaceutical composition according to the third aspect of the present application.

In some preferred embodiments, said biological tissue is a population of cardiomyocytes from a progeria patient.

Compared with the prior art, the present application has at least the following advantages:
The present application designs various polynucleotide sequences targeting the 3' untranslated region (3'UTR) of Lamin A mutant mRNA, which can reduce the expression level of Lamin A mutants (e.g., progerin) at the mRNA stage, while not affecting the transcription of Lamin C, thereby resulting in reduced toxicity. These sequences show broad prospects in the prevention or treatment of diseases associated with lamin A mutations, especially progeria.

It should be understood that, within the scope of the present application, the aforementioned technical features of the present application and the various technical features specifically described below (e.g., in the examples) can be combined with each other to constitute new or preferred technical solutions. For the sake of brevity, they are not exhaustively listed here.

### BRIEF DESCRIPTION OF THE DRAWINGS

The present application is exemplarily illustrated by one or more examples and the accompanying figures corresponding thereto. These exemplary illustrations do not constitute limitations to the examples.
Figure 1 shows the genotyping results of an HGPS child according to an example of the present application.
Figure 2 shows the progerin expression level in fibroblasts from the child as detected by qRT-PCR according to an example of the present application.
Figure 3 is a schematic diagram of the PBMC reprogramming according to an example of the present application.
Figure 4 shows the iPSC phenotype as detected by IFA according to an example of the present application; ns indicates P>0.05; *, indicates P<0.05; ***, indicates P<0.001.
Figure 5 is a schematic diagram showing the cardiomyocyte differentiation process according to an example of the present application.
Figure 6 shows the mRNA expression levels of related genes at different stages of cardiomyocyte differentiation as detected by qRT-PCR according to an example of the present application.
Figure 7 shows the results of IFA detection of cTnT (observing cardiomyofiber length) on day 11 of cardiomyocyte differentiation according to an example of the present application.
Figure 8 shows the results of IFA detection of Lamin A/C (observing nuclear morphology) on day 11 of cardiomyocyte differentiation according to an example of the present application.
Figure 9 is a schematic diagram of the cardiomyocyte passage process according to an example of the present application.
Figure 10 shows the morphology of cardiomyocytes at different passages observed under a microscope bright field according to an example of the present application.
Figure 11 shows SA-β-Gal senescence staining of cardiomyocytes at different passages according to an example of the present application; the percentage value indicates the positive rate of senescence-stained cells; P (Passage) indicates the passage number.
Figure 12 shows IFA detection of cTnT (observing cardiomyofiber length) in cardiomyocytes at different passages according to an example of the present application; P (Passage) indicates the passage number.
Figure 13 shows IFA detection of Lamin A/C (observing nuclear morphology) in cardiomyocytes at different passages according to an example of the present application; P (Passage) indicates the passage number.
Figure 14 shows the design of ASOs targeting progerin in gapmer form according to an example of the present application.
Figure 15 shows the effect of transfection with ASO_U5-U8 on progerin mRNA expression in c.1822G>A cardiomyocytes as detected by qRT-PCR according to an example of the present application. *** indicates P<0.001.
Figure 16 shows the effect of transfection with ASO_U5-U8 on progerin mRNA expression in c.1824C>T cardiomyocytes as detected by qRT-PCR according to an example of the present application. *** indicates P<0.001.
Figure 17 shows the dose-dependent effect of transfection with ASO_U7 and U9 on progerin mRNA expression in c.1822G>A cardiomyocytes as detected by qRT-PCR according to an example of the present application. *** indicates P<0.001.
Figure 18 shows the dose-dependent effect of transfection with ASO_U7 and U9 on progerin mRNA expression in c.1824C>T cardiomyocytes as detected by qRT-PCR according to an example of the present application. *** indicates P<0.001.
Figure 19 shows the dose-dependent effect of transfection with ASO_U7 and U9 on progerin protein expression in c.1822G>A cardiomyocytes as detected by Western blot according to an example of the present application.
Figure 20 shows the dose-dependent effect of transfection with ASO_U7 and U9 on progerin protein expression in c.1824C>T cardiomyocytes as detected by Western blot according to an example of the present application.
Figure 21 shows the effect of ASO_U7 and U9 on cardiomyocyte fiber length and nuclear morphology in classic c.1824C>T and non-classic c.1822G>A cardiomyocytes as detected by IFA (MNA/C [Lamin A/C] staining method) according to an example of the present application.
Figure 22 shows the effect of ASO_U7 and U9 on cardiomyocyte fiber length and nuclear morphology in classic c.1824C>T and non-classic c.1822G>A cardiomyocytes as detected by IFA (cTnT staining method) according to an example of the present application.
Figure 23 shows the results of SA-β-Gal cell senescence staining according to an example of the present application.
Figure 24 shows the proportion of cardiomyocytes with dysmorphic nuclei according to an example of the present application. *** indicates P<0.001.
Figure 25 shows the proportion of SA-β-Gal staining positive cells. *** indicates P<0.001.
Figure 26 shows the effect of subcutaneous injection of ASO_U7 and U9 on progerin mRNA expression in the heart of LMNA G608G progeria mice as detected by qRT-PCR according to an example of the present application. *** indicates P<0.001;
Figure 27 shows the effect of subcutaneous injection of ASO_U7 and U9 on progerin mRNA expression in the liver of LMNA G608G progeria mice as detected by qRT-PCR according to an example of the present application. *** indicates P<0.001.
Figure 28 shows the effect of subcutaneous injection of ASO_U7 and U9 on progerin protein levels in the heart of LMNA G608G progeria mice as detected by Western blot according to an example of the present application.
Figure 29 shows the effect of subcutaneous injection of ASO_U7 and U9 on progerin protein levels in the liver of LMNA G608G progeria mice as detected by Western blot according to an example of the present application.
Figure 30 shows the effect of subcutaneous injection of ASO_U7 and U9 on alanine aminotransferase (ALT) levels in the liver of LMNA G608G progeria mice as detected by ELISA.
Figure 31 shows the effect of subcutaneous injection of ASO_U7 and U9 on aspartate aminotransferase (AST) levels in the liver of LMNA G608G progeria mice as detected by ELISA.
Figure 32 shows the effect of subcutaneous injection of ASO_U7 and U9 on uric acid (UA) levels in the kidney of LMNA G608G progeria mice as detected by ELISA.
Figure 33 shows the effect of subcutaneous injection of ASO_U7 and U9 on creatinine (Crea) levels in the kidney of LMNA G608G progeria mice as detected by ELISA.
Figure 34 shows the effect of subcutaneous injection of ASO_U7 and U9 on urea nitrogen (Urea) levels in the kidney of LMNA G608G progeria mice as detected by ELISA.
Figure 35 shows the protocol for subcutaneous injection of ASO_U7 and PBS control in a progeria mouse model.
Figure 36 shows body weight changes in male progeria mouse models after subcutaneous injection of ASO_U7 ("Ctrl" indicates PBS control).
Figure 37 shows body weight changes in female progeria mouse models after subcutaneous injection of ASO_U7 ("Ctrl" indicates PBS control).
Figure 38 shows the effect of ASO_U7 injection on progerin mRNA expression in the liver of progeria mouse models.
Figure 39 shows the effect of ASO_U7 injection on progerin mRNA expression in the heart of progeria mouse models.
Figure 40 shows the effect of ASO_U7 injection on progerin mRNA expression in the arteries of progeria mouse models.
Figure 41 shows the effect of ASO_U7 injection on progerin protein expression in the liver of progeria mouse models.
Figure 42 shows the effect of ASO_U7 injection on progerin protein expression in the heart of progeria mouse models.
Figure 43 shows the effect of ASO_U7 injection on progerin protein expression in the arteries of progeria mouse models.
Figure 44 shows H&E staining results of arteries from progeria mouse models injected with ASO_U7.
Figure 45 shows the Masson's trichrome staining results indicating fibrosis levels in arteries of progeria mouse models injected with ASO_U7.
Figure 46 shows the Masson's trichrome staining results indicating fibrosis levels in the heart of progeria mouse models injected with ASO_U7.
Figure 47 shows the Masson's trichrome staining results indicating fibrosis levels in the kidney of progeria mouse models injected with ASO_U7.
Figure 48 shows lifespan test results for male progeria mouse models after subcutaneous injection of ASO_U7.
Figure 49 shows lifespan test results for female progeria mouse models after subcutaneous injection of ASO_U7.

### DETAILED DESCRIPTION

To enable those skilled in the art to better understand and implement the technical solutions of the present application, explanations deemed necessary by the applicant regarding the technical application background, technical application environment, and technical terms that may be involved in the detailed description are provided prior to the examples. It should be understood that these explanations shall not be construed as limitations to the present application.

Mutations in the LMNA gene can lead to abnormal splicing of pre-Lamin A mRNA, resulting in the accumulation of lamin A mutants (e.g., progerin or other Lamin A mutants). This accumulation causes various congenital diseases such as progeria, familial dilated cardiomyopathy, muscular dystrophy, and lipodystrophy syndrome. The applicant has discovered through research that reducing Lamin A mRNA levels while preserving Lamin C mRNA levels in mice results in normal physiological and phenotypic indicators, with no observed pathological symptoms. This suggests that Lamin C can functionally compensate for the reduction of Lamin A in cells. Particularly in certain tissues where the normal expression of Lamin A is relatively low, such as brain tissue, Lamin C is the predominant isoform. Consequently, reducing the expression of lamin A mutants without affecting Lamin C levels maintains the normal function of such tissues while significantly improving disease symptoms and progression.

Based on the aforementioned discovery, the applicant has designed various antisense oligonucleotide sequences targeting the 3' untranslated region (3'UTR) of Lamin A mRNA (e.g., progerin mRNA). They are capable of reducing the expression of Lamin A mutants at the Lamin A mRNA level, and therefore, the antisense oligonucleotide sequences of the present application can be used to prevent or treat diseases directly or indirectly caused by LMNA mutations, including dilated cardiomyopathy, conduction disorders, muscular dystrophy, Charcot-Marie-Tooth disease type 2, familial partial lipodystrophy, striated muscle laminopathy, progeroid syndrome, Emery-Dreifuss muscular dystrophy, insulin resistance syndrome, progeria, limb-girdle muscular dystrophy, congenital muscular dystrophy, and Werner's syndrome. Importantly, these sequences do not affect Lamin C levels, thereby enhancing safety. In a preferred embodiment of the present application, the newly designed antisense oligonucleotide sequences of the present application can reduce the expression of progerin, significantly inhibit progeroid phenotypes, while not affecting Lamin C levels or the normal function of brain tissue. Compared to existing treatment methods, such as Lonafarnib (Zokinvy) or other methods for reducing Lamin A mutants, the present invention offers higher safety and holds broad prospects as a drug for preventing or treating progeria.

### Polynucleotide

The present application relates to a polynucleotide. The polynucleotide of the present application can be in the form of DNA or RNA. The DNA form includes cDNA, genomic DNA, or synthetically produced DNA. The DNA can be single-stranded or double-stranded. The DNA can be a coding strand or a non-coding strand. The polynucleotide of the present application is designed to comprise a fragment complementary to a target sequence within a target gene. As used herein, the term "a polynucleotide/gene comprises a fragment of gene X" means that within the entire sequence of said polynucleotide/gene, besides containing the sequence of gene X, other polynucleotide fragments/gene fragments/bases are also provided before and/or after the gene X sequence. For example, the polynucleotide sequence ATCG-X-CCTC includes gene X as a fragment, and the sequence also includes other fragments such as ATCG and CCTC.

As used herein, the terms "polynucleotide fragment" or "fragment" or "gene fragment" refer to a partial sequence of said polynucleotide/gene. That is, the fragment sequence of the polynucleotide should be partially identical to the original polynucleotide. For example, if the original polynucleotide sequence is AAAGGGTTT, the polynucleotide fragment can be any of AAAGGG, AAAG, AGGGTTT, etc.

As used herein, the term " target site is within XX" or " target sequence is within XX" refers to a nucleotide sequence located in the XX region of the gene, or a fragment thereof. In one embodiment, the target site is within the 3' untranslated region (3'UTR) of Lamin A mRNA or Lamin A mutant mRNA (e.g., progerin mRNA), or a fragment thereof. That is, the target site is the 3' untranslated region (3'UTR) of lamin A mRNA or a fragment thereof, or the target site is the 3' untranslated region (3'UTR) of lamin A mutant mRNA or a fragment thereof. In one embodiment of the present application, the target gene is (i) the sequence as shown in SEQ ID NO: 11; and/or (ii) a polynucleotide sequence formed by substitution, deletion, or addition of one or several nucleotides to the sequence shown in SEQ ID NO: 11. In one embodiment of the present application, the target site is within the sequence as shown in SEQ ID NO: 11.

As used herein, the term "mutation" refers to the substitution, deletion, and/or addition of one or more nucleotides in a polynucleotide sequence, or one or more amino acids in an amino acid sequence. As used herein, the term "mutant" refers to an amino acid sequence obtained by mutating the amino acid sequence of a protein at any position, or an amino acid sequence translated from the mRNA of a mutated protein. In the present application, "Lamin A mutant" refers to an amino acid sequence obtained by substitution, deletion, and/or addition of one or more amino acids at any position in the amino acid sequence of Lamin A, or an amino acid sequence translated from the mutated Lamin A mRNA. In a preferred embodiment of the present application, the "Lamin A mutant" is an amino acid sequence translated from a sequence obtained by mutation in exon 11 and/or exon 12 of lamin A mRNA. In a preferred embodiment of the present application, the "Lamin A mutant" is an amino acid sequence translated from a sequence obtained by mutation at one or more positions in lamin A mRNA selected from: c.1711; c.1713; c.1714; c.1718; c.1733; c.1744; c.1745; c.1748; c.1751; c.1756; c.1762; c.1772; c.1774; c.1786; c.1804; c.1821; c.1822; c.1824; c.1851; c.1868; c.1871; c.1892; c.1904; c.1916; c.1928; c.1930; c.1931; c.1940; c.1960; c.1961; c.1968; c.1968+1; c.1968+2; c.1968+5; and c.1975.

In a preferred embodiment of the present application, the "lamin A mutant" is an amino acid sequence translated from a sequence obtained by mutation at one or more positions in lamin A mRNA selected from: c.1821; c.1822; c.1824; c.1968; c.1968+1; c.1968+1G>A; c.1968+2; c.1968+2; c.1968+5; and c.1968+5. Mutation at one or more of these positions will lead to diseases associated with progerin.

In a preferred embodiment of the present application, the "Lamin A mutant" is an amino acid sequence translated from a sequence obtained by one or more mutations in lamin A mRNA selected from: c.1711A>T; c.1713C>A; c.1714A>T; c.1714insCTGC; c.1718C>T; c.1733A>T; c.1744C>T; c.1745G>A; c.1748C>T; c.1751G>A; c.1756G>A; c.1762T>C; c.1772G>T; c.1774G>A; c.1786G>A; c.1804G>A; c.1821G>A; c.1822G>A; c.1824C>T; c.1851C>T; c.1868C>G; c.1871G>A; c.1892G>A; c.1904G>A; c.1916A>G; c.1928C>A; c.1930C>T; c.1931G>A; c.1940T>G; c.1960C>T; c.1961dup; c.1968G>A; c.1968+1G>A; c.1968+2T>C; c.1968+5G>A; c.1968+5G>C; and c.1975dup. In a preferred embodiment of the present application, the "lamin A mutant" is an amino acid sequence translated from a sequence obtained by one or more mutations in lamin A mRNA selected from: c.1821G>A; c.1822G>A; c.1824C>T; c.1968G>A; c.1968+1G>C; c.1968+1G>A; c.1968+2T>C; c.1968+2T>A; c.1968+5G>A; and c.1968+5G>C.

In a preferred embodiment of the present application, the target gene is (i) the sequence as shown in SEQ ID NO: 1; and/or (ii) a polynucleotide sequence formed by substitution, deletion, or addition of one or more nucleotides to the sequence shown in SEQ ID NO: 1.

In a more preferred embodiment of the present application, the target gene is (i) the sequence as shown in SEQ ID NO: 10; and/or (ii) a polynucleotide sequence formed by substitution, deletion, or addition of one or more nucleotides to the sequence shown in SEQ ID NO: 10. Polynucleotides designed to target the sequence shown in SEQ ID NO: 10 more significantly reduce the expression levels of Lamin A mutant (preferably progerin) mRNA and protein.

As used herein, the terms "sequence complementary" and "reverse sequence complementary" are used interchangeably, referring to a sequence that is complementary to the original polynucleotide sequence and in the opposite direction. For example, if the original polynucleotide sequence is ACTGAAC, its reverse complementary sequence is GTTCAGT. In one embodiment of the present application, the polynucleotide sequence is reverse complementary to the sequence shown in SEQ ID NO: 1. In one embodiment of the present application, the polynucleotide sequence is reverse complementary to a fragment of the sequence shown in SEQ ID NO: 1. In one embodiment of the present application, the polynucleotide sequence is reverse complementary to a polynucleotide sequence formed by substitution, deletion, or addition of one or several nucleotides to the sequence shown in SEQ ID NO: 1. In one embodiment of the present application, the polynucleotide sequence is reverse complementary to a fragment of a polynucleotide sequence formed by substitution, deletion, or addition of one or several nucleotides to the sequence shown in SEQ ID NO: 1. In one embodiment of the present application, the polynucleotide sequence is reverse complementary to the sequence shown in SEQ ID NO: 10. In one embodiment of the present application, the polynucleotide sequence is reverse complementary to a fragment of the sequence shown in SEQ ID NO: 10. In one embodiment of the present application, the polynucleotide sequence is reverse complementary to a polynucleotide sequence formed by substitution, deletion, or addition of one or several nucleotides to the sequence shown in SEQ ID NO: 10. In one embodiment of the present application, the polynucleotide sequence is reverse complementary to a fragment of a polynucleotide sequence formed by substitution, deletion, or addition of one or several nucleotides to the sequence shown in SEQ ID NO: 10.

In a preferred embodiment of the present application, the polynucleotide comprises at least one polynucleotide fragment selected from the group consisting of: (i) the polynucleotide fragments as shown in SEQ ID NOs: 2-9; and (ii) polynucleotide fragments having at least 80% (preferably at least 90%, more preferably at least 95%, even more preferably at least 98%, and most preferably at least 99%) sequence identity with SEQ ID NOs: 2-9.

In another preferred embodiment of the present application, the polynucleotide is at least one selected from the group consisting of: (i) the polynucleotides as shown in SEQ ID NOs: 2-9; and (ii) polynucleotides having at least 80% (preferably at least 90%, more preferably at least 95%, even more preferably at least 98%, and most preferably at least 99%) sequence identity with SEQ ID NOs: 2-9.

In another preferred embodiment of the present application, the polynucleotide is at least one selected from the group consisting of: (i) the polynucleotides as shown in SEQ ID NOs: 3-7; and (ii) polynucleotides having at least 80% (preferably at least 90%, more preferably at least 95%, even more preferably at least 98%, and most preferably at least 99%) sequence identity with SEQ ID NOs: 3-7. Compared to other polynucleotide sequences, these polynucleotide sequences exhibit higher knockdown efficiency for lamin A mutant (preferably progerin) mRNA and lamin A mutant protein.

In another preferred embodiment of the present application, the polynucleotide is the polynucleotide as shown in SEQ ID NO: 5; or the polynucleotide as shown in SEQ ID NO: 7. The polynucleotides shown in SEQ ID NO: 5 and 7 have good specificity and low toxicity, reducing the expression levels of progerin mRNA and protein in cardiomyocytes by at least 50% (more preferably at least 70%, and most preferably at least 80%) without targeting other genomic regions, making them suitable for subsequent human trials.

In the present application, the polynucleotide is preferably provided in an isolated form, more preferably purified to homogeneity. As used herein, the term "isolated" refers to a nucleic acid separated from at least one other component (e.g., another nucleic acid) with which it is associated in its natural source. In one embodiment, the nucleic acid is found, if at all, only in solvents, buffers, ions, or other components normally present in its solution. The terms "isolated" and "purified" do not include nucleic acids present in their natural source.

As used herein, the term "identity" refers to the percentage of identical (i.e., the same) nucleotides or amino acids between two or more polynucleotides or polypeptides. Sequence identity between two or more polynucleotides or polypeptides can be measured by the following method. The nucleotide or amino acid sequences of the polynucleotides or polypeptides are aligned, and the number of positions in the aligned polynucleotides or polypeptides that contain identical nucleotide or amino acid residues is scored relative to the number of positions containing different nucleotide or amino acid residues. Polynucleotides can differ at a position, for example, by containing a different nucleotide (i.e., a substitution or variation) or by a deletion of a nucleotide (i.e., an insertion or deletion of one or two nucleotides in the polynucleotide). Polypeptides can differ at a position, for example, by containing a different amino acid (i.e., a substitution or variation) or by a deletion of an amino acid (i.e., an insertion or deletion of one or two amino acids in the polypeptide). Sequence identity can be calculated by dividing the number of positions containing identical nucleotide or amino acid residues by the total number of nucleotide or amino acid residues in the polynucleotide or polypeptide. For example, percent identity can be calculated by dividing the number of positions containing identical nucleotide or amino acid residues by the total number of nucleotide or amino acid residues in the polynucleotide or polypeptide, then multiplying by 100.

In the present application, the aforementioned polynucleotide or fragments thereof can be prepared using conventional methods known in the art, such as PCR amplification, recombination, or artificial synthesis. For PCR amplification, primers can be designed based on publicly known nucleotide sequences, especially the open reading frame sequence, and commercially available cDNA libraries or cDNA libraries prepared by conventional methods known to those skilled in the art can be used as templates for amplification. When the sequence is long, two or more rounds of PCR amplification are often required, and then the amplified fragments from each round are spliced together in the correct order. Once the relevant sequence is obtained, it can be produced in large quantities by recombination. This typically involves cloning it into a vector, introducing it into cells, and then isolating the relevant sequence from the proliferated host cells by conventional methods. Artificial synthesis is used to synthesize the relevant sequence, especially when the fragment length is short. Typically, sequences of considerable length can be obtained by first synthesizing several small fragments and then ligating them.

Regarding the length of the polynucleotide in the present application, it is preferably at least 10 bp, further preferably at least 15 bp, more preferably 18-22 bp, for example, 20 bp, with 20 bp being the most effective length.

### Small Nucleic Acid Molecule

The present application also relates to a small nucleic acid molecule comprising the aforementioned polynucleotide. These small nucleic acid molecules may be siRNA, ASO, shRNA, or miRNA; preferably ASO.

As used herein, the term "antisense oligonucleotide" or "ASO" refers to a polynucleotide or fragment thereof that is complementary to a specific target gene according to the principle of base complementarity, to inhibit or block the expression of the specific target gene. The target gene can be a DNA or RNA sequence. To enhance the stability, target specificity, and metabolic capabilities of the antisense oligonucleotide, the antisense oligonucleotide herein may also include chemical modifications, such as the oxygen atom of the phosphoester bond being modified by phosphorothioate, the 2' position of the ribose being modified by an alkyl group (e.g., a methyl group), Locked Nucleic Acid (LNA) modification, and the like. To promote cellular uptake or improve organ/tissue targeting, the ASO herein may also include terminal cholesterol (Chol) or GalNAc modifcation. In the ASO of the present application, the polynucleotide or fragment complementary to the target gene may optionally also include, at its front end and/or back end, 1-5 (more preferably 3-5) short sequences composed of any bases. The bases in these short sequences are preferably chemically modified. Those skilled in the art can obtain the antisense oligonucleotide of the present application by conventional methods in the art, such as chemical synthesis.

As used herein, the term "siRNA" (Small interfering RNA) refers to a short RNA molecule (typically 21-25 nucleotides) that can be processed by Dicer (an enzyme in the RNAse III family specific for double-stranded RNA) from its precursor (such as dsRNA, shRNA, etc.), or can be produced by other proteins or via chemical synthesis. siRNA is a key component of the siRISC, which triggers the rapid cleavage and degradation of the target RNA complementary to its sequence, leading to silencing of the target gene. Given a specific gene target site sequence, those skilled in the art can design and obtain siRNA targeting that site by conventional methods.

As used herein, the term "miRNA" (microRNA) refers to a class of endogenous, non-coding single-stranded RNA molecules (typically 20-24 nt in length), involved in regulating gene expression. Most miRNA genes exist in the genome as single copies, multiple copies, or gene clusters. Each miRNA can regulate multiple target genes, and several miRNAs can also jointly regulate the same gene, forming a complex regulatory network. miRNAs exist in multiple forms during biogenesis, the primary transcript (pri-miRNA) is processed by Drosha to become a precursor (pre-miRNA, typically 50-90 nt); the pre-miRNA is further cleaved by Dicer enzyme to become mature miRNA (20-24 nt). miRNA typically inhibits target gene expression by inhibiting translation and/or accelerating mRNA deadenylation, a mechanism different from siRNA-mediated mRNA degradation.

One method for producing siRNA in vivo is to clone the sequence as part of a "short hairpin" into a plasmid vector. When delivered into an animal, the hairpin sequence is expressed, forming a "short hairpin RNA" (shRNA) with a loop structure at the top, which is recognized and processed by the intracellular Dicer protein to produce functional siRNA.

As used herein, the precursor serves as a backbone for constructing a special type of shRNA. Said shRNA comprises, from the 5' end to the 3' end: (a) a 5' flanking sequence region; (b) a 5' paired siRNA region; (c) a top loop region; (d) a 3' paired siRNA region, wherein said 5' paired siRNA region and said 3' paired siRNA region form a double-stranded region; (e) a 3' flanking sequence region; said shRNA produces siRNA, and the nucleotide sequence of said siRNA corresponds to said 3' paired siRNA region or 5' paired siRNA region.

Broadly, shRNA is an abbreviation for "short hairpin RNA". shRNA comprises two short reverse complementary sequences separated by a loop sequence, forming a hairpin structure, typically transcribed under the control of an endogenous RNA polymerase III promoter in the cell, and the end of the shRNA sequence is linked to 5-6 thymidines as transcription terminator for RNA polymerase III. shRNA can also be transcribed from other RNA polymerase promoters.

### Pharmaceutical Composition

The present application also relates to a pharmaceutical composition containing the polynucleotide of the present application. The pharmaceutical composition of the present application comprises the polynucleotide of the present application and a pharmaceutically acceptable carrier. To better enable precise delivery of the polynucleotide of the present application to specific sites in a subject, or to improve the bioavailability of the polynucleotide relative to the subject, or to reduce the toxicity of the polynucleotide relative to the subject, the pharmaceutically acceptable carrier may optionally be a liposome, lipid nanoparticle (LNP), protamine, polymer (e.g., polyethyleneimine), inorganic nanoparticle, exosome or polymer matrix, etc.

In other preferred embodiments of the present application, the pharmaceutical composition comprises: the polynucleotide of the present application and a lipid nanoparticle shell encapsulating it. Preferably, the lipid nanoparticle shell is composed of at least one component selected from a PEG lipid, cholesterol, ionizable cationic lipid, and helper lipid. To increase targeting, the lipid nanoparticle shell can optionally be modified with surface modifiers.

In other preferred embodiments of the present application, the polynucleotide sequence is conjugated to an asialoglycoprotein receptor ligand (GalNAc) to form GalNAc-X, where X represents the polynucleotide of the present application.

### Use or Indication

The present application also relates to the use of the polynucleotide or pharmaceutical composition for (i) preventing and/or treating a disease associated with a LMNA mutation; (ii) preparing a medicament for preventing and/or treating a disease associated with a LMNA mutation; (iii) reducing the expression level of a Lamin A mutant; (iv) reducing the number of senescence-positive cells; and/or (v) reducing the number of cells with dysmorphic nuclei.

In the present application, any mRNA sequence capable of specifically binding to the polynucleotide sequence of the present application can be cleaved by ribonuclease H1, leading to reduced expression levels of the protein translated from that mRNA. Therefore, the polynucleotide sequence of the present application targets the 3'UTR region of lamin A mutant mRNA, causing degradation of Lamin A mutant mRNA and thereby reducing the expression level of the Lamin A mutant, thus enabling the treatment of diseases associated with Lamin A mutations.

As used herein, the term "disease associated with a Lamin A mutation" may include any disease directly or indirectly caused by a mutation in Lamin A gene or or Lamin A mRNA. Preferably, the Lamin A mutation is a mutation occurring in exon 11 and/or exon 12 of Lamin A mRNA. The disease associated with a lamin A mutation is preferably a disease caused by a mutation in exon 11 and/or exon 12 of lamin A mRNA. In a preferred embodiment of the present application, the "disease associated with a Lamin A mutation" is a disease caused by a mutation in Lamin A mRNA at one or more positions selected from the following: c.1711; c.1713; c.1714; c.1718; c.1733; c.1744; c.1745; c.1748; c.1751; c.1756; c.1762; c.1772; c.1774; c.1786; c.1804; c.1821; c.1822; c.1824; c.1851; c.1868; c.1871; c.1892; c.1904; c.1916; c.1928; c.1930; c.1931; c.1940; c.1960; c.1961; c.1968; c.1968+1; c.1968+2; c.1968+5; and c.1975. The aforementioned mutation positions can be queried on the website http://www.umd.be/LMNA/. In a preferred embodiment of the present application, the "disease associated with a Lamin A mutation" is a disease caused by one or more mutations in Lamin A mRNA selected from: c.1711A>T; c.1713C>A; c.1714A>T; c.1714insCTGC; c.1718C>T; c.1733A>T; c.1744C>T; c.1745G>A; c.1748C>T; c.1751G>A; c.1756G>A; c.1762T>C; c.1772G>T; c.1774G>A; c.1786G>A; c.1804G>A; c.1821G>A; c.1822G>A; c.1824C>T; c.1851C>T; c.1868C>G; c.1871G>A; c.1892G>A; c.1904G>A; c.1916A>G; c.1928C>A; c.1930C>T; c.1931G>A; c.1940T>G; c.1960C>T; c.1961dup; c.1968G>A; c.1968+1G>A; c.1968+2T>C; c.1968+5G>A; c.1968+5G>C; and c.1975dup. For example, the disease associated with a Lamin A mutation is selected from at least one of: dilated cardiomyopathy and conduction disorders, muscular dystrophy, Charcot-Marie-Tooth disease type 2, familial partial lipodystrophy, striated muscle laminopathy, progeroid syndrome, Emery-Dreifuss muscular dystrophy, insulin resistance syndrome, progeria, limb-girdle muscular dystrophy, congenital muscular dystrophy, and Werner's syndrome, preferably progeria.

### Therapeutic Method

The present application also relates to a method for preventing and/or treating a disease associated with a Lamin A mutation, comprising the step of: administering to a subject in need thereof a therapeutically effective amount of the polynucleotide of the present application, or a pharmaceutical composition of the present application. Regarding the mode of administration, the polynucleotide of the present application may be administered directly as an ASO or in the form of a pharmaceutical composition.

As used herein, the term "subject" is defined herein to include animals, particularly a mammal, including but not limited to, primates (e.g., humans), cows, sheep, goats, horses, dogs, cats, rabbits, rats, mice, etc. In specific embodiments, the subject is a human.

As used herein, the term "therapeutically effective amount" refers to an amount of an active ingredient sufficient to provide a therapeutic effect in the treatment or control of a disease or disorder, or sufficient to delay or minimize one or more symptoms associated with the disease or disorder. The therapeutically effective amount of a compound refers to the amount of a therapeutic agent that, when used alone or in combination with other therapies, provides a therapeutic effect in the treatment or control of a disease or disorder. The term "therapeutically effective amount" can include an amount that improves the overall therapy, reduces or avoids the symptoms or causes of the disease or disorder, or enhances the therapeutic efficacy of another therapeutic agent. In a preferred embodiment of the present application, the administration dosage of the polynucleotide or pharmaceutical composition of the present application is preferably 1 to 500 mg/kg (when the subject is human), for example, 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1 mg/kg, 1.5 mg/kg, 2 mg/kg, 2.5 mg/kg, 3 mg/kg, 3.5 mg/kg, 4.5 mg/kg, 5 mg/kg, 5.1 mg/kg, 5.2 mg/kg, 5.3 mg/kg, 5.4 mg/kg, 5.5 mg/kg, 5.6 mg/kg, 5.7 mg/kg, 5.8 mg/kg, 5.9 mg/kg, 6 mg/kg, 7 mg/kg, 8 mg/kg, 9 mg/kg, 10 mg/kg, 20 mg/kg, 30 mg/kg, 40 mg/kg, 50 mg/kg, or 100 mg/kg. When the subject is another non-human mammal, the corresponding administration dosage can be determined based on dose conversion methods well known in the art.

The administration dosage of the polynucleotide or pharmaceutical composition of the present application is preferably 1 to 500 mg/kg (when the subject is a mouse), for example, 10 mg/kg, 20 mg/kg, 30 mg/kg, 40 mg/kg, 50 mg/kg, or 100 mg/kg.

As used herein, the term "treating" refers to eradicating or improving a disease or condition, or one or more symptoms associated with the disease or condition. In one embodiment, it is known to those skilled in the art that such symptoms are associated with the disease or condition to be treated. In specific embodiments, the term refers to minimizing the progression or worsening of a disease or condition by administering one or more prophylactic or therapeutic agents to a subject suffering from the disease or condition. In some embodiments, the term refers to administering the polynucleotide of the present application, with or without other additional active agents, after the onset of symptoms of a specific disease.

As used herein, the term "preventing" refers to preventing the onset, recurrence, or spread of a disease or condition, or one or more symptoms associated with the disease or condition. In one embodiment, it is known to those skilled in the art that such symptoms are associated with the disease or condition to be prevented. In specific embodiments, the term refers to administering the polynucleotide provided herein, with or without other additional active agents, to a patient at risk of developing the disease or disorder described herein, before the onset of symptoms. The term includes the suppression and reduction of symptoms of a specific disease. In specific embodiments, patients with a family history of a disease are specifically targeted as candidates. Furthermore, patients with a history of recurrent symptoms are also potential candidates for prevention. In this regard, the term "preventing" can be used interchangeably with the term "prophylactic treatment".

The route of administration of the polynucleotide or pharmaceutical composition of the present application is not limited, and is preferably via intramuscular, subcutaneous, oral, intravenous, dermal, mucosal (e.g., enteral), intranasal, or intraperitoneal routes.

The frequency of administration of the polynucleotide or pharmaceutical composition of the present application is preferably once daily, twice daily, three times daily, once every two days, once every three days, once every five days, once a week, twice a week, once a month, twice a month, three times a month, once every two months, once every three months, once every four months, once every five months, once every six months, or once a year.

### Other In Vitro Application Methods

The present application also relates to a non-therapeutic in vitro method for (i) reducing the expression level of a Lamin A mutant, or (ii) reducing the number of senescence-positive cells, or (iii) reducing the number of cells with dysmorphic nuclei, comprising the step of contacting a biological tissue with the polynucleotide of the present application in a non-therapeutic manner in vitro. As an example of a non-therapeutic application, it can be applied to scientific research and laboratory drug screening, among other areas.

In some preferred schemes, the biological tissue is a population of cardiomyocytes from a patient with progeria. In one embodiment of the present application, when the polynucleotide of the present application is contacted in vitro with a population of cardiomyocytes from a progeria patient, the expression level of the Lamin A mutant (e.g., progerin) is significantly decreased. In one embodiment of the present application, when the polynucleotide of the present application is contacted in vitro with a population of cardiomyocytes from a progeria patient, the number of senescence-positive cells is significantly reduced. In one embodiment of the present application, when the polynucleotide of the present application is contacted in vitro with a population of cardiomyocytes from a progeria patient, the number of cells with dysmorphic nuclei is significantly reduced. In one embodiment of the present application, when the polynucleotide of the present application is contacted in vitro with a population of cardiomyocytes from a progeria patient, the expression level of progerin is significantly reduced.

To make the objectives, technical solutions, and advantages of the embodiments of the present application clearer, the present application is further described in detail below with reference to specific Examples. It should be understood that these Examples are only used to illustrate the present application and are not intended to limit the scope of the present application. The experimental methods using unspecified specific conditions in the following Examples are generally carried out under conventional conditions, or under conditions suggested by the manufacturer. Unless otherwise stated, percentages and parts are by weight. The experimental materials and reagents used in the following Examples can be obtained from commercial sources unless otherwise specified.

Unless otherwise indicated, the technical and scientific terms used herein have the same meaning as commonly understood by one of ordinary skill in the art to which this application belongs. It should be noted that the terms used herein are for describing specific embodiments only and are not intended to limit the exemplary embodiments of the present application.

### EXAMPLE 1

### Genotyping of Multi-genotype HGPS and Reprogramming to Obtain iPSCs

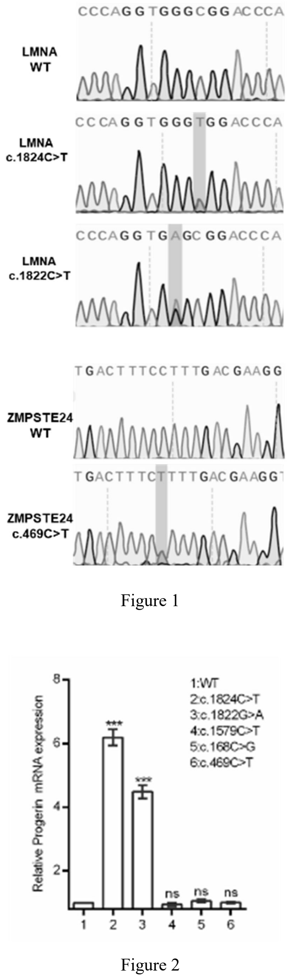

Peripheral blood mononuclear cells (PBMCs) were collected and isolated from an HGPS child patient. Genomic DNA was extracted, and the LMNA and ZMPSTE24 genes were amplified. The patients' pathogenic genotype was determined by comparison with the genome of a normal individual. Most patients had the genotype of classical HGPS (c.1824C>T). Non-classical LMNA mutation HGPS genotypes, such as c.1579C>T, c.1822G>A, and c.168C>G, as well as the ZMPSTE24 mutation type c.469C>T, were also detected (Fig. 1). The expression of progerin mRNA in the patient was detected by qRT-PCR. The results showed high expression of aberrantly spliced LMNA mRNA (encoding the progerin protein) in both classical c.1824C>T and non-classical c.1822G>A cells (Fig. 2). Finally, we selected patients with the classical c.1824C>T, non-classical c.1822G>A, and ZMPSTE24 mutation c.469C>T genotypes for subsequent studies.

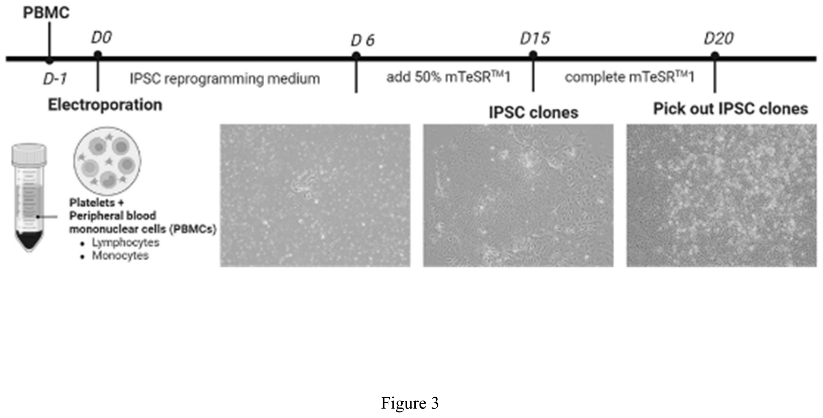

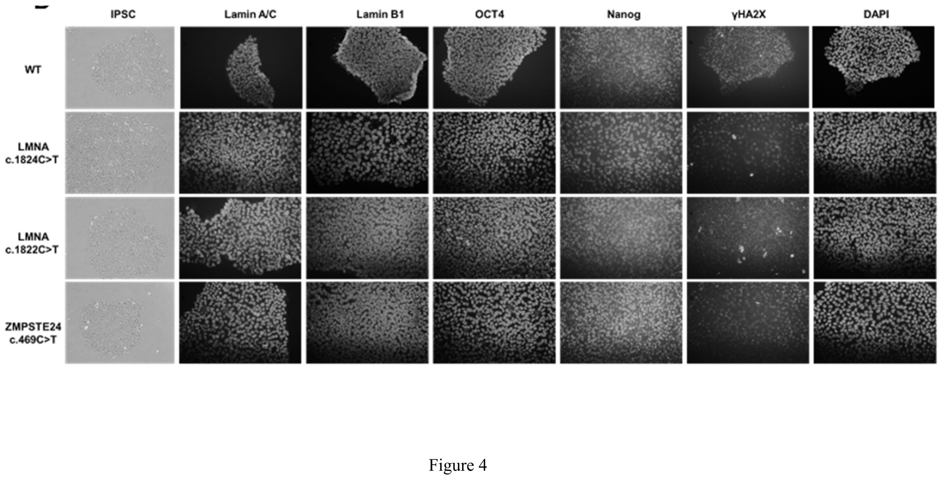

To obtain iPSCs of the above genotypes, the reprogramming plasmids pCXLE-hSK, pCXLE-hUL, and pCXLE-hOCT3/4 were electroporated into PBMCs isolated from the patient's blood. iPSC single clones were picked on day 20 (Fig. 3). The obtained iPSCs were passaged continuously, and their karyotype and stemness were detected by immunofluorescence assay (IFA) (Fig. 4). The results showed that PBMCs from various HGPS genotypes could be reprogrammed into iPSCs. After continuous passaging, the karyotype and stemness of the cells showed no significant difference compared to normal human iPSCs. HGPS patient-derived iPSCs did not exhibit significant DNA damage (p-γH2AX staining), consistent with previous reports indicating that iPSCs at the HGPS stage do not show an obvious senescence phenotype.

### EXAMPLE 2

### Construction and Characterization of HGPS Cardiomyocyte Differentiation Model

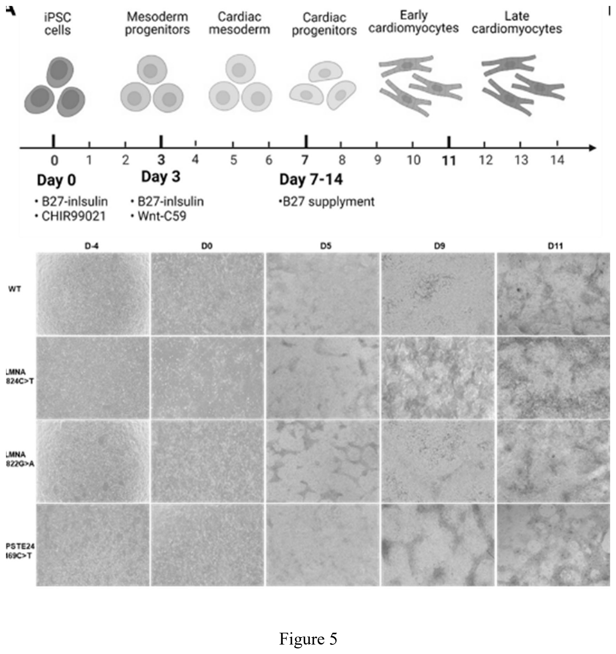

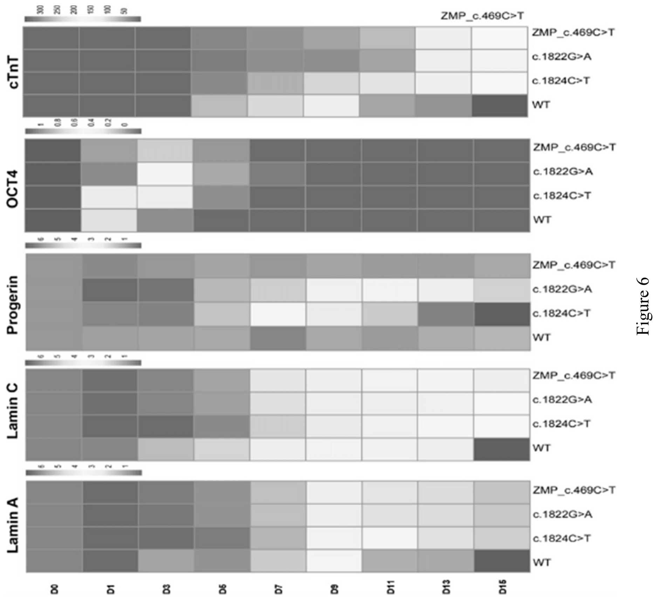

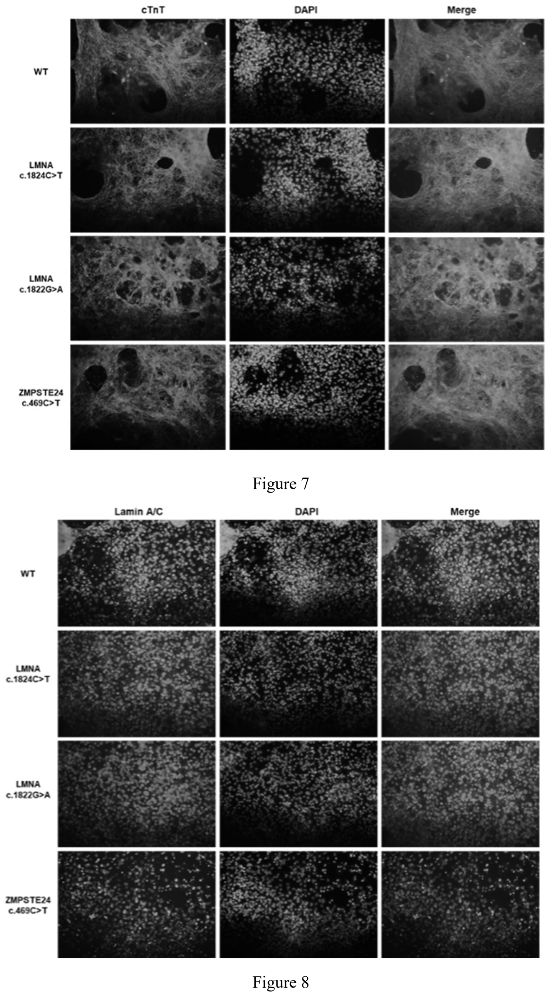

Cardiomyocyte differentiation from iPSCs can be efficiently induced by modulating the Wnt pathway. By optimizing initial cell density, the concentration of the GSK-3 inhibitor CHIR99021, and the concentration of the Wnt pathway inhibitor Wnt-C59, a method was established for differentiating cardiomyocytes from multiple HGPS genotypes (Fig. 5). Partial cell beating was observed on day 7 of differentiation (cardiac progenitor stage), and by day 9, at least 95% of the cells exhibited rhythmic, synchronized beating. qRT-PCR detection revealed that progerin expression began in c.1824C>T and c.1822G>A cells on day 7 and continued to increase with prolonged differentiation time (Fig. 6). Concurrently, expression of the stemness marker gene OCT4 decreased, while the expression of cardiac troponin T (cTnT) increased rapidly starting on day 5. Statistical analysis of cardiomyocyte beating frequency showed that the beating frequency in the WT group was significantly higher than that in the HGPS groups. IFA staining for Lamin A/C and cTnT showed no obvious nuclear deformation or difference in myofiber length in HGPS cells during the early stages of cardiomyocyte differentiation (Figs. 7 and 8).

### EXAMPLE 3

### Phenotypic Identification and Senescence Marker Detection in Passaged HGPS Cardiomyocytes

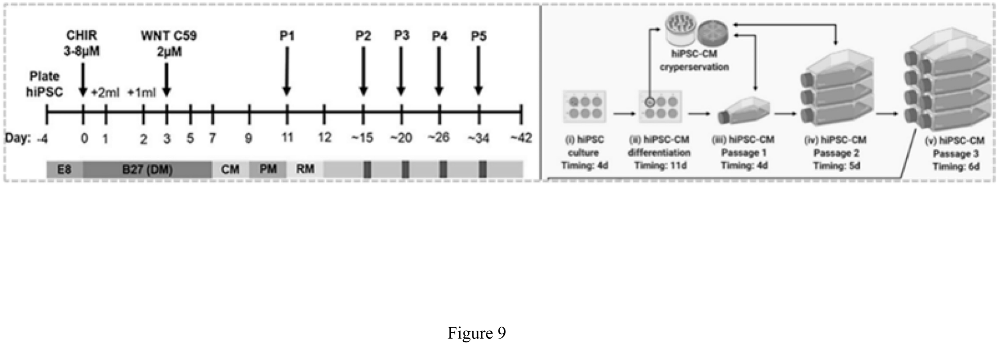

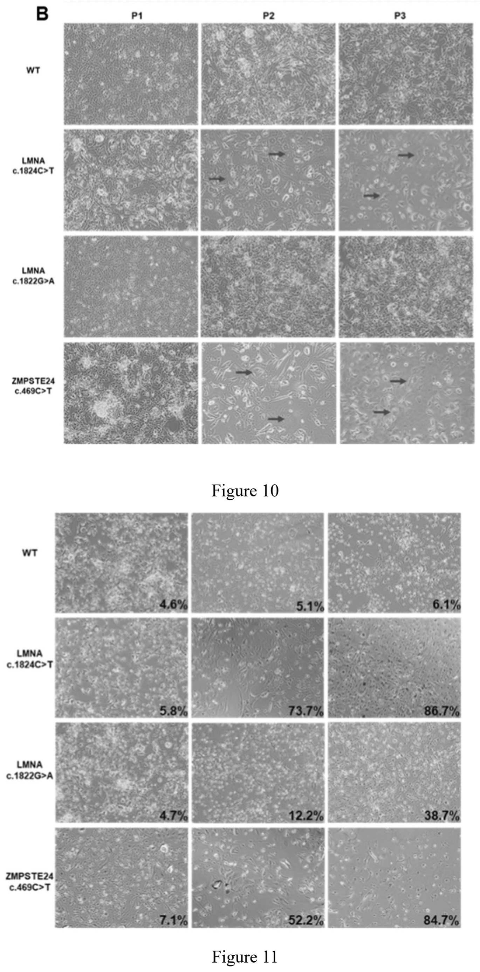

To investigate phenotypic changes during the passaging of cardiomyocytes from different HGPS genotypes, the differentiated cardiomyocytes were continuously passaged. Senescence was assessed using methods such as SA-β-Gal staining, qRT-PCR, immunofluorescence analysis, beating frequency measurement, and cell proliferation assays. The results showed that compared to the wild-type cells, cardiomyocytes from different HGPS genotypes exhibited varying degrees of senescence phenotype with increasing passage number, with the classical c.1824C>T phenotype being particularly pronounced (Figs. 9 to 13).

### EXAMPLE 4

### Design of ASOs Targeting Progerin

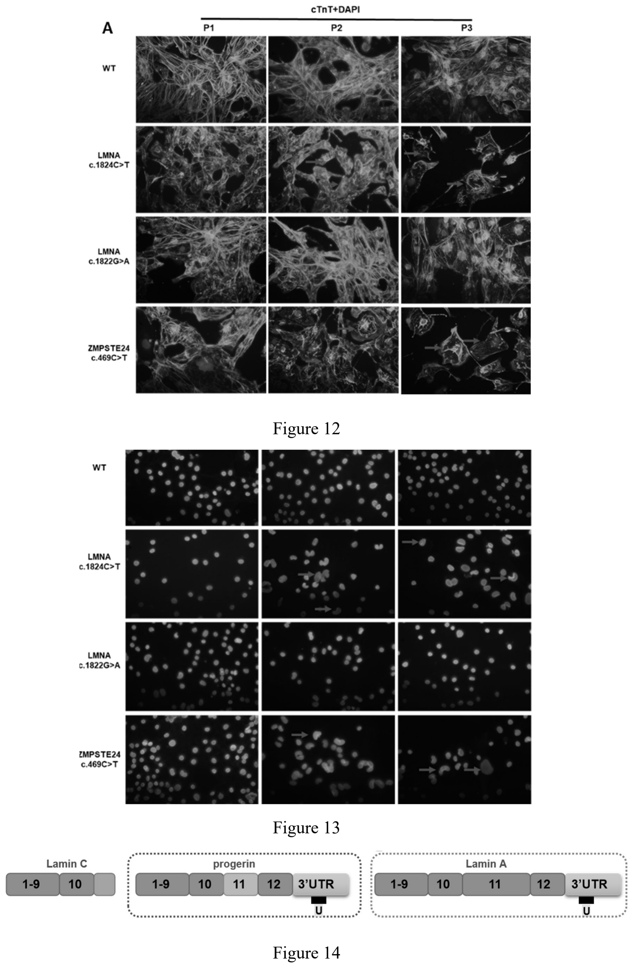

ASOs represent a promising approach for nucleic acid drug development. In view of the limitations of existing small-molecule therapies such as Lonafarnib (Zokinvy), ASOs represent an alternative option for clinical treatment of HGPS. In this example, several ASO sequences targeting the 3'UTR were designed, with the 3'UTR sequence shown in SEQ ID NO: 11. These ASOs are capable of reducing the expression level of the progerin protein by targeting progerin mRNA, thereby mitigating the damage caused by progerin to cells and slowing the aging process (a schematic diagram of the progerin-targeting ASO design is shown in Fig. 14).

First, suitable target gene sequences for ASO design were identified. These target sequences are fragments of the 3'UTR, such as the sequence shown in SEQ ID NO: 1.
SEQ ID NO: 1: CACATCTGCCTTAAAACCAAAGAGGGCTT

Furthermore, a sequence suitable for ASO design targets the sequence shown in SEQ ID NO: 10.
SEQ ID NO: 10: CATCTGCCTTAAAACCAAAGAGGGC
SEQ ID NO: 11:

ATCTGGGACCTGCCAGGCAGGGGTGGGGGTGGAGGCTTCCTGCGTCCTCC
TCACCTCATGCCCACCCCCTGCCCTGCACGTCATGGGAGGGGGCTTGAAGC
CAAAGAAAAATAACCCTTTGGTTTTTTTCTTCTGTATTTTTTTTTCTAAGAG
AAGTTATTTTCTACAGTGGTTTTATACTGAAGGAAAAACACAAGCAAAAAA
AAAAAAAAGCATCTATCTCATCTATCTCAATCCTAATTTCTCCTCCCTTCCTT
TTCCCTGCTTCCAGGAAACTCCACATCTGCCTTAAAACCAAAGAGGGCTTC
CTCTAGAAGCCAAGGGAAAGGGGTGCTTTTATAGAGGCTAGCTTCTGCTTT
TCTGCCCTGGCTGCTGCCCCCACCCCGGGGACCCTGTGACATGGTGCCTGA
GAGGCAGGCATAGAGGCTTCTCCGCCAGCCTCCTCTGGACGGCAGGCTCA
CTGCCAGGCCAGCCTCCGAGAGGGAGAGAGAGAGAGAGAGGACAGCTTG
AGCCGGGCCCCTGGGCTTGGCCTGCTGTGATTCCACTACACCTGGCTGAGG
TTCCTCTGCCTGCCCCGCCCCCAGTCCCCACCCCTGCCCCCAGCCCCGGGG
TGAGTCCATTCTCCCAGGTACCAGCTGCGCTTGCTTTTCTGTATTTTATTTAG
ACAAGAGATGGGAATGAGGTGGGAGGTGGAAGAAGGGAGAAGAAAGGTG
AGTTTGAGCTGCCTTCCCTAGCTTTAGACCCTGGGTGGGCTCTGTGCAGTC
ACTGGAGGTTGAAGCCAAGTGGGGTGCTGGGAGGAGGGAGAGGGAGGTC
ACTGGAAAGGGGAGAGCCTGCTGGCACCCACCGTGGAGGAGGAAGGCAA
GAGGGGGTGGAGGGGTGTGGCAGTGGTTTTGGCAAACGCTAAAGAGCCCT
TGCCTCCCCATTTCCCATCTGCACCCCTTCTCTCCTCCCCAAATCAATACAC
TAGTTGTTTCTACCCCT

The applicant designed multiple ASO sequences targeting the 3'UTR fragment as listed in Table 1 below, and commissioned a company to synthesize the corresponding fragments. The synthesized fragments were then dissolved in sterile water for subsequent use. Cardiomyocytes differentiated from iPSCs of progeria patients with different genotypes were evenly plated in 6-well plates at a density of 5×10⁵ cells/well. After 48 hours, when cardiomyocyte beating was observed, the cells were prepared for ASO transfection. Using Lipofectamine 3000 transfection reagent, different ASOs were transfected into the respective cardiomyocytes at concentrations of 50 nM or 100 nM. A mock cell group and a transfection reagent negative control group were also established. After transfection, the cardiomyocytes were cultured for an additional 48 hours before cell sample collection. For RNA sample collection: The supernatant in the 6-well plates was aspirated and discarded, and the cells were gently washed twice with PBS. Then, 350 µL of TRK cell lysis buffer was added to each well. Total cellular RNA was extracted using the OMEGA RNA Extraction Kit (R6824-02) and stored for subsequent detection. For protein sample collection: The supernatant in the 6-well plates was aspirated and discarded, and the cells were gently washed twice with PBS. Then, 200 µL of Beyotime RIPA Lysis Buffer (P0013B) was added to each well. The cells were lysed on ice for 15 minutes, followed by centrifugation at 4°C and 12,000 rpm. The supernatant was collected, and the protein concentration was measured before storage for subsequent detection. Real-time quantitative PCR (qPCR) was performed to detect the expression of progerin and other mRNAs. Detection primers were designed based on the sequences of the progerin and other target genes. Total RNA was reverse transcribed into cDNA using the Vazyme Reverse Transcription Kit (R223-01). The expression levels of progerin and other mRNAs were then quantified using the Vazyme SYBR-based Fluorescent Quantitative Kit (Q711-03). Statistical data were plotted accordingly. For Western blot detection of protein expression: Protein electrophoresis was performed using ACE Gradient Protein Separation Gel (B221212) with a sample load of 30 µg protein per well, at 160 V for 90 minutes. Semi-dry transfer was then conducted. After incubation with the corresponding antibodies, protein bands were visualized using a chemiluminescence imager, and protein expression levels were recorded.

**Table 1**

| SEQ ID | ASO name | Sequence information |
|---|---|---|
| SEQ ID NO: 2 | U4 | TTGGTTTTAAGGCAGATGTG |
| SEQ ID NO: 3 | U5 | CTTTGGTTTTAAGGCAGATG |
| SEQ ID NO: 4 | U6 | CTCTTTGGTTTTAAGGCAGA |
| SEQ ID NO: 5 | U7 | CCTCTTTGGTTTTAAGGCAG |
| SEQ ID NO: 6 | U8 | CCCTCTTTGGTTTTAAGGCA |
| SEQ ID NO: 7 | U9 | GCCCTCTTTGGTTTTAAGGC |
| SEQ ID NO: 8 | U10 | AGCCCTCTTTGGTTTTAAGG |
| SEQ ID NO: 9 | U11 | AAGCCCTCTTTGGTTTTAAG |

### EXAMPLE 5

### Validation of ASO Efficacy in Reducing Progerin mRNA Expression

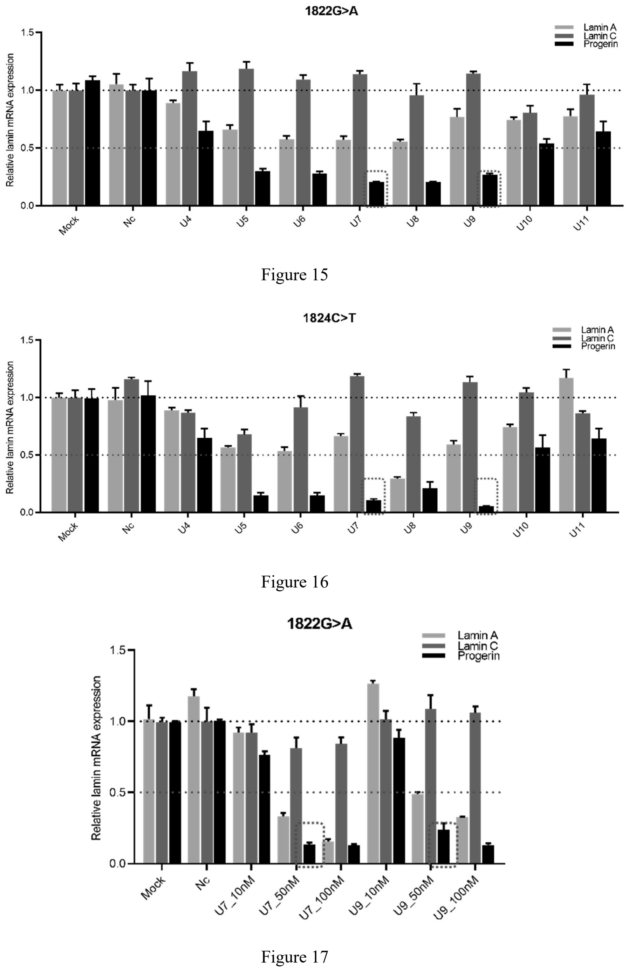

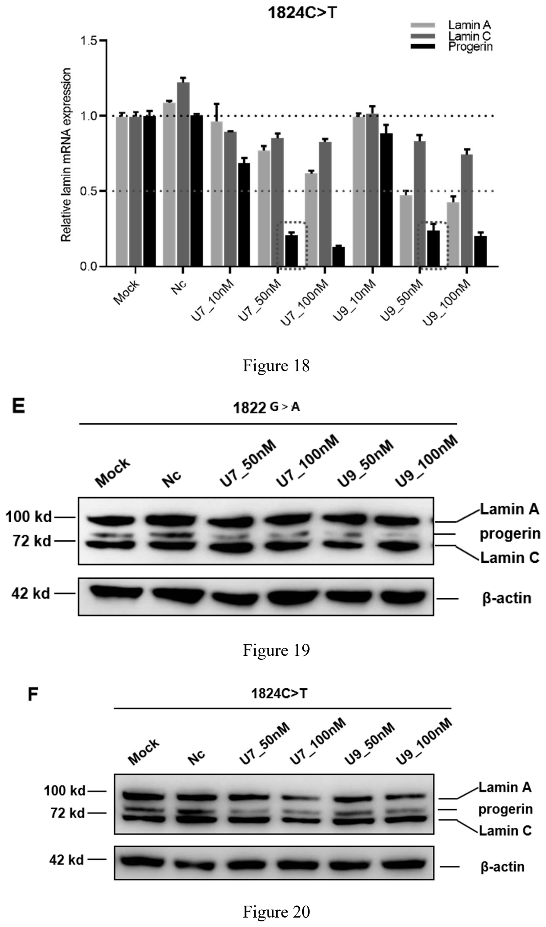

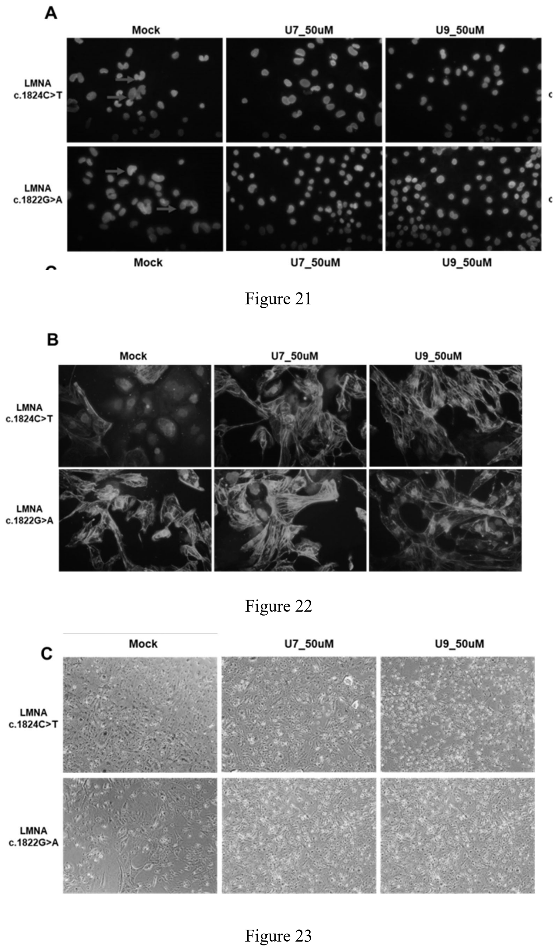

To investigate the effects of different ASOs in the progeria cardiomyocyte model, the inventors transfected various types of ASOs into classical c.1824C>T and non-classical c.1822G>A cardiomyocytes. qRT-PCR results showed that ASO_U5-U11 (100 nM) could all significantly reduce progerin mRNA expression in cardiomyocytes, with ASO_U5-U9 reducing progerin mRNA expression by more than 70% (Figs. 15 and 16). Taking ASO_U7 and ASO_U9 as examples, dose-dependence experiments proved that 50 nM ASO_U7 and ASO_U9 effectively reduced progerin mRNA and protein expression levels (Figs. 17-20). IFA analysis showed a significant reduction in the number of dysmorphic nuclei after transfection with ASO_U7 and U9. SA-β-Gal staining results also confirmed that transfection with U7 and U9 reduced the number of senescence-positive cells (Figs. 21-25). The above results demonstrate that the progerin-targeting ASO_U7 and U9 can significantly reduce progerin mRNA expression and slow the cellular senescence process.

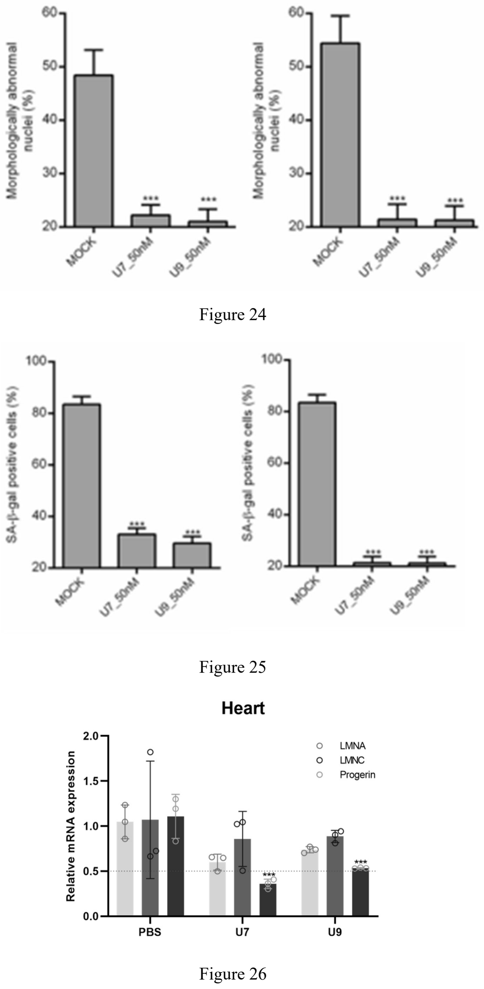

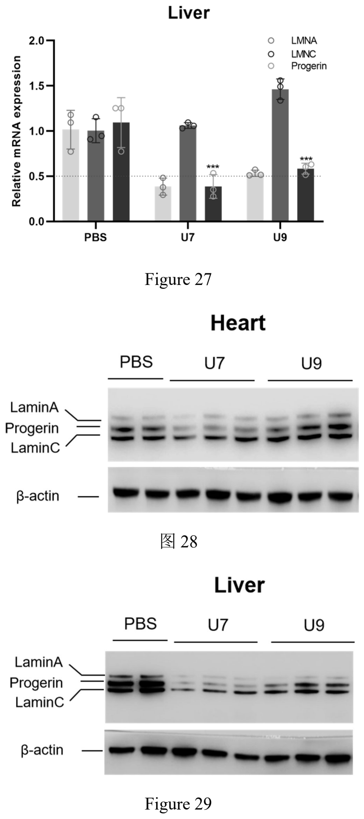

To further assess the effects of ASOs in a progeria mouse model, we screened the above-designed ASO sequences. In the LMNA G608G (c.1824C>T mutation) progeria mouse model, we subcutaneously injected ASO_U7 and U9 (50 mg/kg) separately once per week, with a PBS group as the control. After one month of continuous injection, the expression of progerin mRNA and protein in the heart and liver tissues of mice was detected, and certain inhibitory effect was found on the expression of progerin mRNA and protein (for example, as shown in Figs. 26-29, qRT-PCR and Western blot results showed that both ASO_U7 and ASO_U9 could significantly reduce the expression of progerin mRNA and protein in the heart and liver of progeria mice).

### EXAMPLE 6

### Specificity and Toxicity of ASOs

In the study, it was found that the specificity of U7 and U9 designed in the above examples was better than that of U5, U6, and U8, and in particular, U7 had the best specificity.

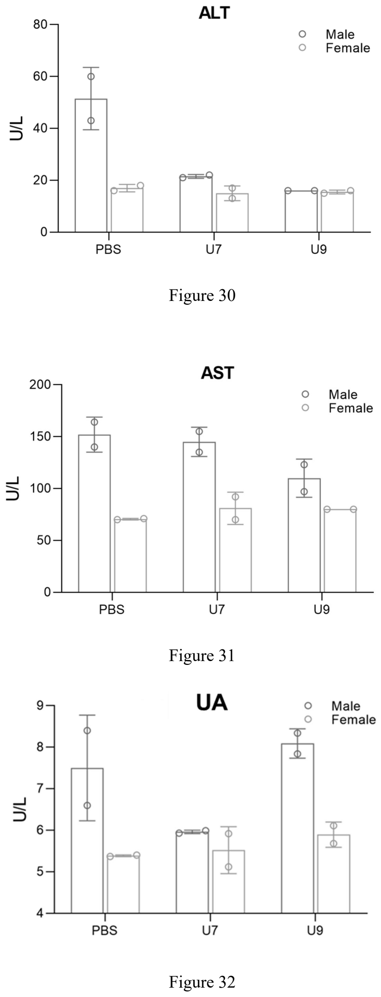

The inventors collected mouse serum from the PBS, ASO_U7, and U9 injection groups, and performed detection of biochemical indicators related to liver and kidney, respectively. Through experiments, it was found that ASO_U7 had the best specificity and the lowest toxicity, making it more suitable for subsequent human trials. For example, as shown in Figs. 30-34, compared with the PBS injection group, the ASO_U7 group would not cause significant liver and kidney damage.

### EXAMPLE 7

### Effects of ASO on the Progeria Mouse Model

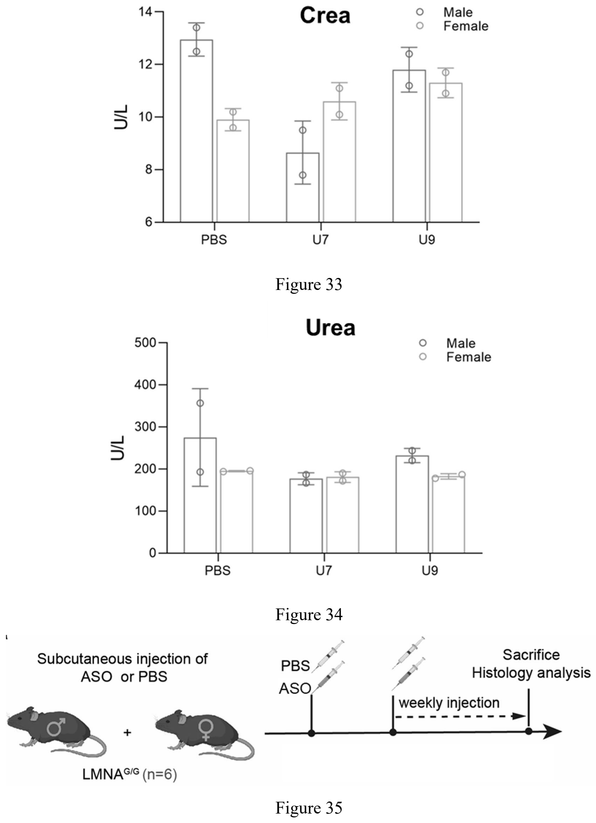

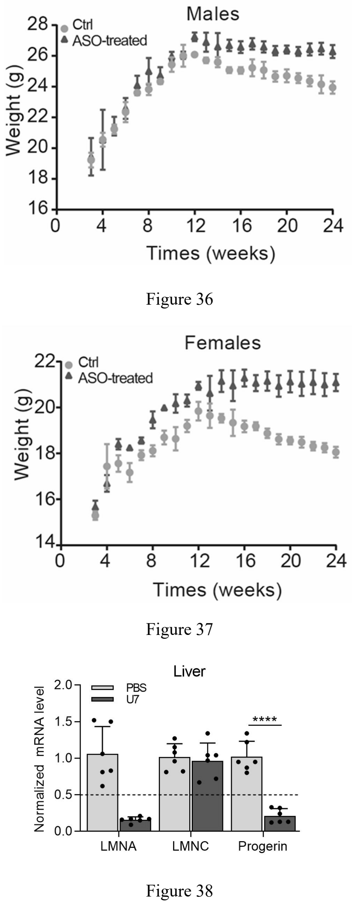

To further investigate the effects of ASO on the progeria mouse model, we tested the above-designed ASO_U7. In the LMNA G608G progeria mouse model (3 males, 3 females), ASO_U7 (50 mg/kg) was subcutaneously injected once per week, as shown in Fig. 35, with a PBS group as the control. Injections were continued for five months, and body weight was weighed and recorded weekly before each injection (Figs. 36 and 37). After five months of injection, mice were euthanized, and liver, heart, and arterial tissues of the mice were collected under low-temperature conditions for detection of progerin mRNA and protein expression in various tissues and organs of the mice, respectively. At the same time, heart, arterial, and kidney tissues and organs of the mice were collected at room temperature, fixed with 4% paraformaldehyde, and subjected to histopathological examination (H&E staining and Masson staining). The results showed that the body weight of the mice in the ASO_U7 injection group was significantly higher than in the PBS control group (Figs. 36 and 37), indicating that ASO_U7 could significantly improve and reverse the weight loss phenomenon in progeria mice.

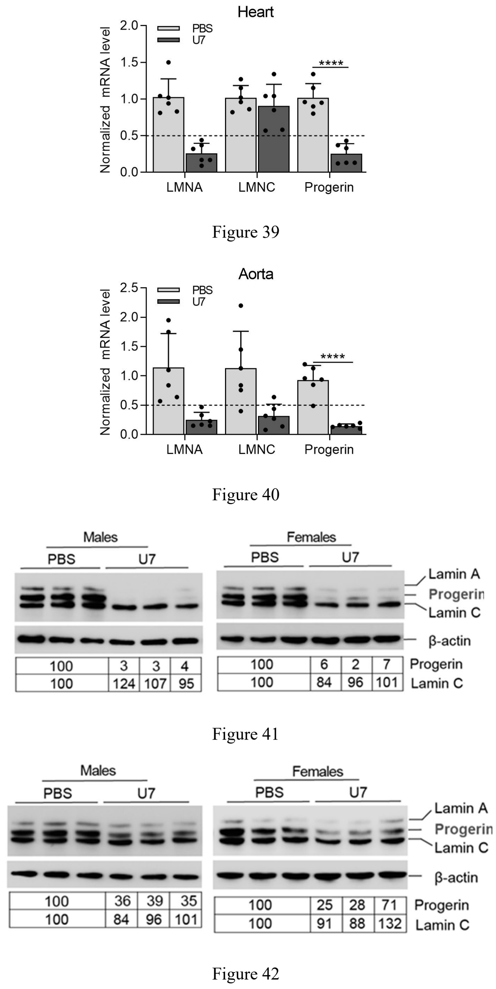

mRNA detection results showed that ASO_U7 could significantly inhibit progerin mRNA expression in the heart, liver, and arteries of mice (Figs. 38-40). Protein detection results showed that ASO_U7 effectively inhibited progerin protein expression in the liver, heart, and arteries of mice (Figs. 41-43).

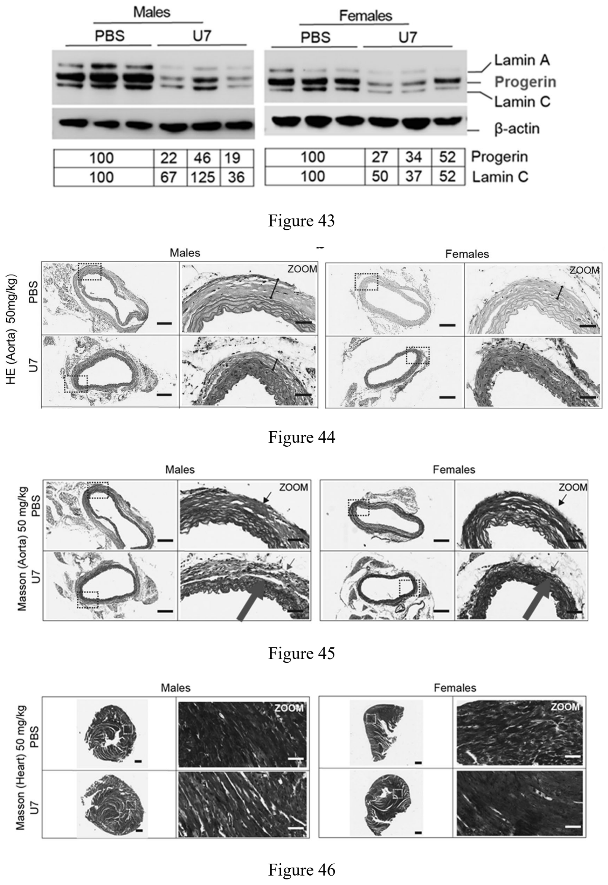

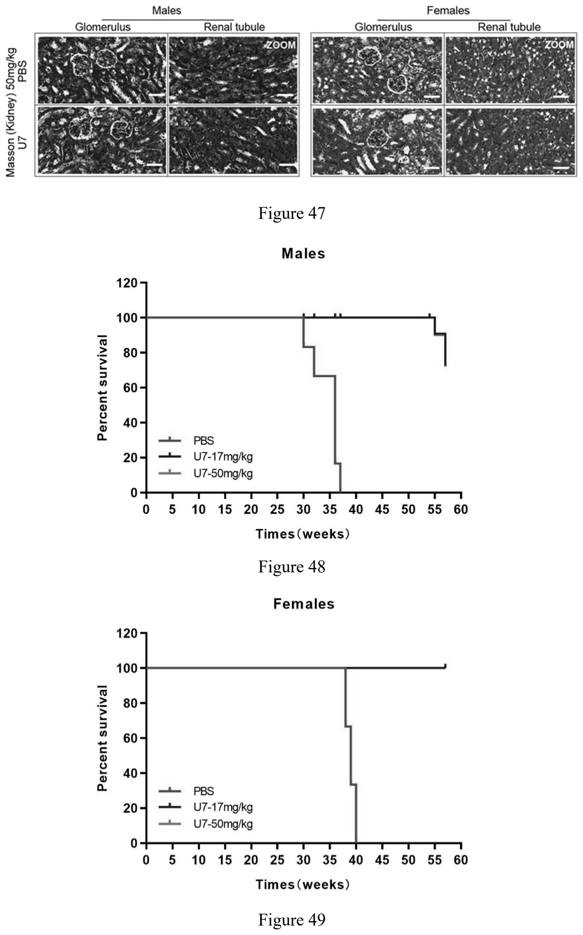

Masson staining results showed that injection of ASO_U7 effectively reduced fibrosis levels in arteries (Fig. 45), heart (Fig. 46), and kidneys (Fig. 47) of both male and female progeria mice.

### EXAMPLE 8

### Effect of ASO on the Lifespan of the Progeria Mouse Model

To investigate the effect of ASO on the lifespan of the progeria mouse model, we tested ASO_U7, and we subcutaneously injected ASO_U7 at 17 mg/kg and 50 mg/kg, respectively, once a week in the LMNA G608G progeria mouse model (6 males, 6 females) (injection procedure as shown in Fig. 35), with a PBS group as the control. Mouse mortality was monitored continuously, and lifespan was recorded. The lifespan test results showed that injection of ASO_U7 extended the lifespan of both male (Fig. 48) and female (Fig. 49) progeria mice by at least three months, or even longer.

It will be understood by those skilled in the art that the foregoing embodiments are specific examples for implementing the present application. In practical applications, various changes can be made in form and detail without departing from the spirit and scope of the present application.

In further embodiments, the assessment of risk is based on the measurement of an expression level of a specific gene panel comprising at least one gene, and up to 203 genes, selected from a curated set of markers associated with endometrial non-receptivity. These markers may include, without limitation, FOSB, IGHG2, IGHG3, IGLC2, CXCL8, CXCL9, IFNG, IL1B, SERPINB3, S100A8, and XCL1. The expression levels may be quantified through various molecular biology techniques, such as RNA-seq, quantitative PCR, or mass spectrometry. An increased risk is determined if the expression level of one or more of these genes exceeds a reference threshold characteristic of successful implantation outcomes.

### Nucleotide and/or amino acid sequence listing

<110> Organization Name: Liangzhu lab
   Application Project
<120> Title: Polynucleotide, Pharmaceutical Composition and Uses Thereof
<130> AppFileReference: None
<140> CurrentAppNumber:
   <141> CurrentFilingDate: ___-__-__
   Earlier Applications
<150> PriorAppNumber: 2023106881238
   <151> PriorFilingDate: 2023-06-09
   Sequence
   <213> Organism Name : Artificial Sequence 1
<400> PreSequenceString: CACATCTGCCTTAAAACCAAAGAGGGCTT 29 <212> Type : DNA
   <211> Length : 29
   SequenceName : SEQ ID NO: 1
   SequenceDescription :
   <213> OrganismName : Artificial Sequence 2
<400> PreSequenceString : TTGGTTTTAAGGCAGTGTG 20 <212> Type : DNA
   <211> Length : 20
   SequenceName : SEQ ID NO: 2
   SequenceDescription :
   <213> OrganismName : Artificial Sequence 3
<400> PreSequenceString : CTTTGTTTTAAGGCAGATG 20 <212> Type : DNA
   <211> Length : 20
   SequenceName : SEQ ID NO: 3
   <213> OrganismName : Artificial Sequence 4
<400> PreSequenceString : CTCTTTGGTTTTAAGGCAGA 20 <212> Type : DNA
   <211> Length : 20
   SequenceName : SEQ ID NO: 4
   <213> OrganismName : Artificial Sequence 5
<400> PreSequenceString : CCTCTTTGGTTTTAAGGCAG 20 <212> Type : DNA
   <211> Length : 20
   SequenceName : SEQ ID NO: 5
   <213> OrganismName : Artificial Sequence 6
<400> PreSequenceString : CCCTCTTTGGTTTTAAGGA 20 <212> Type : DNA
   <211> Length : 20
   SequenceName : SEQ ID NO: 6
   <213> OrganismName : Artificial Sequence 7
<400> PreSequenceString : GCCCTCTTTGGTTTTAAGGC 20 <212> Type : DNA
   <211> Length : 20
   SequenceName : SEQ ID NO: 7
   <213> OrganismName : Artificial Sequence 8
<400> PreSequenceString : AGCCCTCTTTGGTTTTAAGG 20
<212> Type : DNA
   <211> Length : 20
   SequenceName : SEQ ID NO: 8
   <213> OrganismName : Artificial Sequence 9
<400> PreSequenceString : AAGCCCTCTTTGGTTTTAAG 20 <212> Type : DNA
   <211> Length : 20
   SequenceName : SEQ ID NO: 9
   <213> OrganismName : Artificial Sequence 10
<400> PreSequenceString : CATCTGGCCTTAAAACCAAAGAGGGC 25 <212> Type : DNA
   <211> Length : 25
   SequenceName : SEQ ID NO: 10
   <213> OrganismName : Artificial Sequence 11
<400> PreSequenceString : 
ATCTGGGACC TGCCAGGCAG GGGTGGGGGT GGAGGCTTCC 40
TGCGTCCTCC TCACCTCATG CCCACCCCCT GCCCTGCACG 80
TCATGGGAGG GGGCTTGAAG CCAAAGAAAA ATAACCCTTT 120
GGTTTTTTTC TTCTGTATTT TTTTTTCTAA GAGAAGTTAT 160
TTTCTACAGT GGTTTTATAC TGAAGGAAAA ACACAAGCAA 200
AAAAAAAAAA AAGCATCTAT CTCATCTATC TCAATCCTAA 240
TTTCTCCTCC CTTCCTTTTC CCTGCTTCCA GGAAACTCCA 280
CATCTGCCTT AAAACCAAAG AGGGCTTCCT CTAGAAGCCA 320
AGGGAAAGGG GTGCTTTTAT AGAGGCTAGC TTCTGCTTTT 360
CTGCCCTGGC TGCTGCCCCC ACCCCGGGGA CCCTGTGACA 400
TGGTGCCTGA GAGGCAGGCA TAGAGGCTTC TCCGCCAGCC 440
TCCTCTGGAC GGCAGGCTCA CTGCCAGGCC AGCCTCCGAG 480
AGGGAGAGAG AGAGAGAGAG GACAGCTTGA GCCGGGCCCC 520
TGGGCTTGGC CTGCTGTGAT TCCACTACAC CTGGCTGAGG 560
TTCCTCTGCC TGCCCCGCCC CCAGTCCCCA CCCCTGCCCC 600
CAGCCCCGGG GTGAGTCCAT TCTCCCAGGT ACCAGCTGCG 640
CTTGCTTTTC TGTATTTTAT TTAGACAAGA GATGGGAATG 680
AGGTGGGAGG TGGAAGAAGG GAGAAGAAAG GTGAGTTTGA 720
GCTGCCTTCC CTAGCTTTAG ACCCTGGGTG GGCTCTGTGC 760
AGTCACTGGA GGTTGAAGCC AAGTGGGGTG CTGGGAGGAG 800
GGAGAGGGAG GTCACTGGAA AGGGGAGAGC CTGCTGGCAC 840
CCACCGTGGA GGAGGAAGGC AAGAGGGGGT GGAGGGGTGT 880
GGCAGTGGTT TTGGCAAACG CTAAAGAGCC CTTGCCTCCC 920
CATTTCCCAT CTGCACCCCT TCTCTCCTCC CCAAATCAAT 960
ACACTAGTTG TTTCTACCCCT 981
 <212> Type : DNA
   <211> Length : 981
   Sequence Name: SEQ ID NO: 11

## Claims

1. A polynucleotide, **characterized in that** the length of said polynucleotide is not less than 10 bp, and said polynucleotide comprises a polynucleotide fragment complementary to the following sequence or a fragment thereof:
(i) the sequence as shown in SEQ ID NO: 11; and/or
(ii) a polynucleotide sequence formed by substitution, deletion, or addition of one or several nucleotides to the sequence shown in SEQ ID NO: 11.

2. The polynucleotide according to claim 1, **characterized in that** said polynucleotide comprises a polynucleotide fragment complementary to the following sequence or a fragment thereof:
(i) the sequence as shown in SEQ ID NO: 1; and/or
(ii) a polynucleotide sequence formed by substitution, deletion, or addition of one or several nucleotides to the sequence shown in SEQ ID NO: 1.

3. The polynucleotide according to claim 1, **characterized in that** said polynucleotide comprises a polynucleotide fragment complementary to the following sequence or a fragment thereof:
(i) the sequence as shown in SEQ ID NO: 10; and/or
(ii) a polynucleotide sequence formed by substitution, deletion, or addition of one or several nucleotides to the sequence shown in SEQ ID NO: 10.

4. The polynucleotide according to claim 1, **characterized in that** said polynucleotide comprises at least one polynucleotide fragment selected from the group consisting of:
(i) the polynucleotide fragments as shown in SEQ ID NOs: 2-9; and
(ii) polynucleotide fragments having at least 80% sequence identity with SEQ ID NOs: 2-9.

5. The polynucleotide according to claim 1, **characterized in that** said polynucleotide is at least one selected from the group consisting of:
(i) the polynucleotides as shown in SEQ ID NOs: 2-9; and
(ii) polynucleotides having at least 80% sequence identity with SEQ ID NOs: 2-9.

6. The polynucleotide according to claim 5, **characterized in that** said polynucleotide is the polynucleotide as shown in SEQ ID NO: 5 or the polynucleotide as shown in SEQ ID NO: 7.

7. The polynucleotide according to any one of claims 1-6, **characterized in that** said polynucleotide is obtained by isolation.

8. A pharmaceutical composition, **characterized in that** said pharmaceutical composition comprises the polynucleotide according to any one of claims 1 to 6, and a pharmaceutically acceptable carrier.

9. Use of polynucleotide according to any one of claims 1 to 6 or the pharmaceutical composition according to claim 8, **characterized in that** said use comprises at least one of the following:
(i) preventing and/or treating a disease associated with a Lamin A mutation;
(ii) preparing a medicament for preventing and/or treating a disease associated with a Lamin A mutation;
(iii) reducing the expression level of a Lamin A mutant;
(iv) reducing the number of senescence-positive cells;
(v) reducing the number of cells with dysmorphic nuclei.

10. The use according to claim 9, **characterized in that** said Lamin A mutation is a mutation in a region of the LMNA gene corresponding to exon 11 and/or exon 12.

11. The use according to claim 9, **characterized in that** said disease associated with a Lamin A mutation is a disease caused by a mutation occurring in exon 11 and/or exon 12 of Lamin A mRNA.

12. The use according to claim 11, **characterized in that** said disease associated with a lamin A mutation is a disease caused by a mutation in lamin A mRNA at one or more positions selected from: c.1711; c.1713; c.1714; c.1718; c.1733; c.1744; c.1745; c.1748; c.1751; c.1756; c.1762; c.1772; c.1774; c.1786; c.1804; c.1821; c.1822; c.1824; c.1851; c.1868; c.1871; c.1892; c.1904; c.1916; c.1928; c.1930; c.1931; c.1940; c.1960; c.1961; c.1968; c.1968+1; c.1968+2; c.1968+5; and c.1975.

13. The use according to claim 12, **characterized in that** said disease associated with a lamin A mutation is a disease caused by one or more mutations in Lamin A mRNA selected from: c.1711A>T; c.1713C>A; c.1714A>T; c.1714insCTGC; c.1718C>T; c.1733A>T; c.1744C>T; c.1745G>A; c.1748C>T; c.1751G>A; c.1756G>A; c.1762T>C; c.1772G>T; c.1774G>A; c.1786G>A; c.1804G>A; c.1821G>A; c.1822G>A; c.1824C>T; c.1851C>T; c.1868C>G; c.1871G>A; c.1892G>A; c.1904G>A; c.1916A>G; c.1928C>A; c.1930C>T; c.1931G>A; c.1940T>G; c.1960C>T; c.1961dup; c.1968G>A; c.1968+1G>A; c.1968+2T>C; c.1968+5G>A; c.1968+5G>C; and c.1975dup.

14. The use according to claim 9, **characterized in that** said disease associated with a lamin A mutation is at least one selected from the group consisting of: dilated cardiomyopathy and conduction disorders (DCM-CD), muscular dystrophy, Charcot-Marie-Tooth disease type 2 (CMT2), familial partial lipodystrophy (FPLD), striated muscle laminopathy, progeroid syndrome, Emery-Dreifuss muscular dystrophy (EDMD), insulin resistance syndrome (IRS), progeria, limb-girdle muscular dystrophy (LGMD), congenital muscular dystrophy (L-CMD) and Werner's syndrome.

15. A small nucleic acid molecule, **characterized in that** said small nucleic acid molecule comprises the polynucleotide according to any one of claims 1-6 or claim 7.

16. The small nucleic acid molecule according to claim 15, **characterized in that** said small nucleic acid molecule is at least one selected from siRNA, ASO, shRNA, and miRNA.

17. The nucleic acid sequence of claim 14, **characterized in that**,

18. A method for preventing and/or treating a disease associated with a lamin A mutation, **characterized in that** the method comprises the step of:
administering a therapeutically effective amount of the polynucleotide according to any one of claims 1 to 6, or the pharmaceutical composition according to claim 8, or the small nucleic acid molecule according to any one of claims 15-17 to a subject.

19. A non-therapeutic in vitro method for reducing the expression level of a lamin A mutant, or reducing the number of senescence-positive cells, or reducing the number of cells with dysmorphic nuclei, **characterized in that** the method comprises the step of:
contacting a biological tissue in a non-therapeutic manner in vitro with the polynucleotide according to any one of claims 1 to 6, or the pharmaceutical composition according to claim 8, or the small nucleic acid molecule according to any one of claims 15-17.

20. The method according to claim 19, **characterized in that** said biological tissue is a population of cardiomyocytes derived from progeria patients.
